# EUROPEAN PATENT APPLICATION

(11) **EP 2 818 548 A1**
(43) Date of publication of application: **31.12.2014**
(21) Application number: 13751778.5
(22) Date of filing: 19.02.2013
(51) Int. Cl.: C12N 15/09, A01H 5/00, A01H 5/02, C12N 1/15, C12N 1/19, C12N 1/21, C12N 5/10

(54) **TORENIA-ORIGINATED PROMOTER CAPABLE OF ACTING IN PETALS**

(30) Priority: 24.02.2012 JP 2012038644
(71) Applicant: Suntory Holdings Limited, Osaka-shi, Osaka 530-8203 (JP)
(72) Inventor: TANAKA, Yoshikazu, Mishima-gun Osaka 618-8503 (JP); KATSUMOTO, Yukihisa, Mishima-gun Osaka 618-8503 (JP)
(74) Representative: Keirstead, Tanis Evelyne
(86) International application number: PCT/JP2013/054017
(87) International publication number: WO 2013/125530

(57) **Abstract**

A novel promoter useful for altering the color of flowers of a plant, which is selected from the group consisting of: (1) a nucleic acid which comprises the nucleotide sequence represented by SEQ ID NO: 20, SEQ ID NO: 12 or SEQ ID NO: 21; and (2) a nucleic acid which maintains the same promoter activity as that of the nucleotide sequence represented by SEQ ID NO: 20, SEQ ID NO: 12 or SEQ ID NO: 21, and which comprises a nucleotide sequence that is produced by modifying the original nucleotide sequence by the addition, deletion and/or substitution of one or several nucleotide sequences, or can hybridize with a nucleotide acid comprising a nucleotide sequence complementary to the original nucleotide sequence under highly stringent conditions, or has a 90% or more sequence identity to the original nucleotide sequence.

## Description

### TECHNICAL FIELD

The present invention relates to a novel promoter. More particularly, the present invention relates to a transcription regulatory region of flavonoid 3',5'-hydroxylase (abbreviated as F3'5') gene or flavone synthase (abbreviated as FNS) gene derived from torenia, and to the use thereof.

### BACKGROUND ART

New traits can be imparted to plants by expressing a useful gene in a target plant using genetic recombination technology. Transgenic plants produced in this manner are already grown commercially on a wide scale. Since regulation of gene expression is primarily controlled at the transcription level, transcriptional regulation is of the greatest importance in terms of regulating gene expression. Namely, the transcription of a target gene at a suitable time, in a suitable tissue, and at a suitable strength is important for producing an industrially useful transgenic plant. In many cases, the start of transcription is controlled by a DNA sequence on the 5'-side of a translation region. A region of DNA that determines the starting site of the transcription of a gene and directly regulates the frequency thereof is referred to as a promoter. Promoters are present at a location several bp from the 5'-side of a start codon, and frequently include a sequence such as a TATA box. Cis-regulatory elements that bind various transcription regulatory factors are further present on the 5'-side, and the presence of these elements controls such factors as the timing of transcription, the tissue where transcription takes place or the strength of transcription. Transcription regulatory factors are classified into numerous families according to their amino acid sequences. Examples of some more well-known families include Myb-type transcription factors and bHLH (basic helix-loop-helix)-type transcription factors. In actuality, transcription control regions and promoters are frequently used in the same context and are not rigidly distinguished.

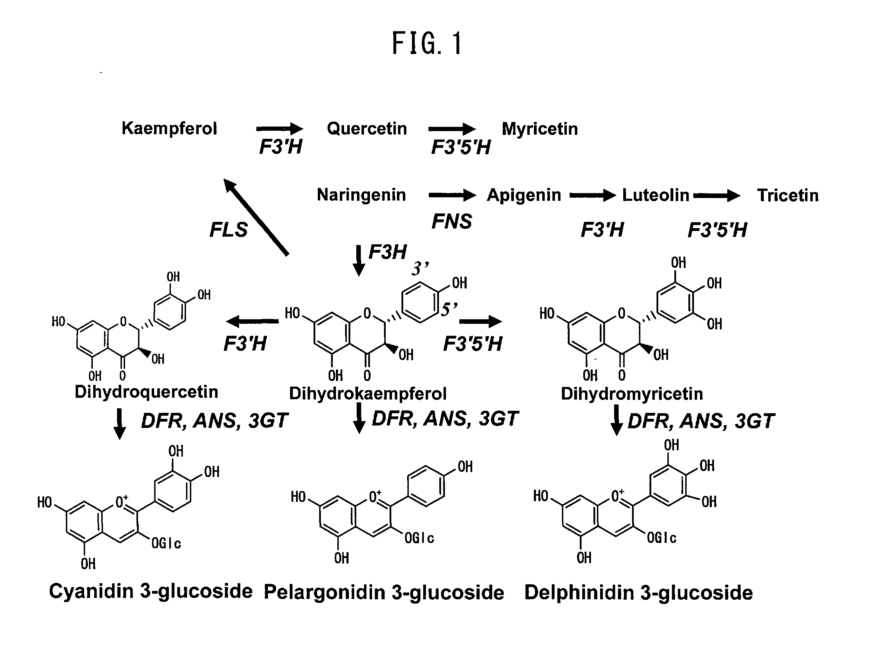

Anthocyanins, which are a main component of flower color, are one of the members of secondary metabolites generally referred to as flavonoids. The color of anthocyanins is dependent upon the structure thereof. Namely, flower color becomes blue with an increase in the number of hydroxyl groups on the B ring of anthocyanidins, which are chromophores of anthocyanins. Typical examples of anthocyanidins include delphinidin, cyanidin and pelargonidin, and the number of hydroxyl groups on the B ring of these compounds is 3, 2 and 1, respectively. Among these, delphinidin results in the bluest color (see FIG. 1). In addition, as the number of aromatic acyl groups (such as coumaroyl groups or caffeoyl groups) that modify an anthocyanin increases, the color produced by the anthocyanin is known to become blue (namely, the absorption maximum shifts towards longer wavelengths) and the stability of the anthocyanin is known to increase. Alternatively, in the case a flavonoid such as a flavone is present with an anthocyanin, the resulting color is known to become bluer and deeper. Such effects are referred to as copigment effects, and flavone is a type of copigment (see Non-Patent Document 1).

Extensive research has been conducted on enzymes involved in anthocyanin synthesis and the gene encoding those enzymes (see Non-Patent Document 1). For example, flavonoid 3'-hydroxylase (abbreviated as F3'H) and F3'5'H are enzymes that catalyze a reaction that increases the number of hydroxyl groups on the B ring. F3'H is required for synthesis of cyanidin while F3'5'H is required for the synthesis of delphinidin. Since delphinidin is not synthesized in such plants as roses, carnations and chrysanthemums, there are no blue varieties of these plants. There are examples of changing the flower color of these plants to blue by expressing F3'5'H gene in these plants (see International Publication No. WO 1996/036716, Patent Document 4 and International Publication No. WO 2009/062253). Flavones are synthesized from flavonoids by catalysis by the enzyme FNS. There are examples of changing the flower color of petunia by expressing flavone synthase gene therein (see Plant Biotechnol., 21, 377-386 (2004)).

Moreover, findings have also been obtained regarding the transcriptional regulation of genes involved in the biosynthesis of anthocyanins. A cis-regulatory element is present in the transcription control region of these genes located on the 5'-side of a start codon that binds with an Myb-type transcription regulatory factor and bHLH-type transcription regulatory factor. Myb-type transcription regulatory factor and bHLH-type transcription regulatory factor are known to control the synthesis of anthocyanins in plants such as petunia, corn and perilla (see Non-Patent Document 1).

In plants, promoters responsible for gene transcription (to also be referred to as transcription regulatory regions) consist of so-called constitutional promoters that function in any tissue and at any time in the developmental stage, organ/tissue-specific promoters that function only in specific organs and tissues, and time-specific promoters that are expressed only at specific times in the developmental stage. Constitutional promoters are frequently used as promoters for expressing useful genes in transgenic plants. Typical examples of constitutional promoters include cauliflower 35S promoter and promoters constructed on the basis thereof (see Non-Patent Document 3) and Mac1 promoter (see Non-Patent Document 4). In plants, however, many genes are expressed organ/tissue-specifically or time-specifically. This indicates that it is necessary for plants that these genes be expressed in specific tissues or organs or at specific times. There are examples of carrying out gene recombination in plants using such tissue/organ-specific or time-specific transcription regulatory regions. For example, protein has been made to accumulate in seeds by using a seed-specific transcription regulatory region.

There are several reports on promoters that function specifically in flower petals or primarily function in flower petals along with their use. For example, there are examples of changing flower color by expressing F3'5'H gene in petunia or carnation using the promoter of chalcone synthase gene derived from snapdragon or petunia. The promoter of rose chalcone synthase gene has been shown to function in rose and chrysanthemum. There are also examples of changing flower color by expressing the promoter of cineraria F3'5'H gene in petunia. In addition, there are also examples of using the flavanone 3-hydroxylase gene promoter of chrysanthemum to express F3'5'H gene in chrysanthemum petals.

However, it is difficult to predict the degree to which a promoter will function and bring about a desired phenotype in a target recombinant plant. In addition, gene silencing may occur if a base sequence identical or similar to a host plant is introduced or if numerous copies of an introduced gene are inserted or that gene is inserted repeatedly (see Non-Patent Document 5). Thus, the repeated use of the same promoter to express a plurality of exogenous genes should be avoided since it can cause gene silencing. In addition, even though the enzyme genes involved in flavonoid synthesis may function in flower petals, the timing of their expression differs. In general, flavones and flavonols are known to be expressed when flowers are younger in comparison with anthocyanins. For example, the acquisition of a plurality of promoters having different expression timing is industrially important for changing flower color.

### Prior Art Documents

### Patent Documents

Patent Document 1: International Publication No. WO96/25500
Patent Document 2: International Publication No. WO01/72984
Patent Document 3: International Publication No. WO94/28140
Patent Document 4: International Publication No. WO05/17147

### Non-Patent Documents

Non-Patent Document 1: Plant J., 54, 737-749, 2008
Non-Patent Document 2: Agricultural and Biological Chemistry, 53, 797-800, 1989
Non-Patent Document 3: Plant Cell Physiology, 1996, 37, 49-59
Non-Patent Document 4: Plant Molecular Biology, 1990, 15, 373-381
Non-Patent Document 5: Annals of Botany, 1997, 79, 3-12

### DISCLOSURE OF THE INVENTION

### Problems to be Solved by the Invention

An object of the present invention is to provide a novel promoter that is useful for changing the flower color of a plant.

### Means for Solving the Problems

As a result of conducting extensive studies to solve the aforementioned problems, the inventors of the present invention discovered that the transcription regulatory regions of torenia-derived F3'5'H gene an FNS gene are novel promoters that are useful for changing the flower color of a plant and confirmed the usefulness thereof, thereby leading to completion of the present invention.

Namely, the present invention is as described below.

[1] A nucleic acid selected from the group consisting of the following:
   (1) a nucleic acid composed of the base sequence shown in SEQ ID NO: 20;
   (2) a nucleic acid able to function as a transcription regulatory region of torenia F3'5'H gene and composed of a base sequence that has been modified by addition, deletion and/or substitution of one or several base sequences in the base sequence shown in SEQ ID NO: 20;
   (3) a nucleic acid able to function as a transcription regulatory region of torenia F3'5'H gene and hybridize under highly stringent conditions with a nucleic acid composed of a base sequence complementary to the base sequence shown in SEQ ID NO: 20; and,
   (4) a nucleic acid able to function as a transcription regulatory region of torenia F3'5'H gene and having at least 90% sequence identity with the base sequence shown in SEQ ID NO: 20.
[2] An expression vector containing the nucleic acid described in [1] above.
[3] The expression vector described in [2] above, containing the base sequence shown in SEQ ID NO: 20.
[4] A non-human host transformed by the expression vector described in [2] or [3] above.
[5] A plant, or a progeny, a portion or a tissue thereof, introduced with the nucleic acid described in [1] above.
[6] The plant, or progeny, portion or tissue thereof, described in [5] above, which is a cut flower.
[7] A processed cut flower obtained by using the cut flower described in [6] above as a raw material.
[8] A nucleic acid selected from the group consisting of the following:
   (1) a nucleic acid composed of the base sequence shown in SEQ ID NO: 12;
   (2) a nucleic acid able to function as a transcription regulatory region of torenia F3'5'H gene and composed of a base sequence that has been modified by addition, deletion and/or substitution of one or several base sequences in the base sequence shown in SEQ ID NO: 12;
   (3) a nucleic acid able to function as a transcription regulatory region of torenia F3'5'H gene and hybridize under highly stringent conditions with a nucleic acid composed of a base sequence complementary to the base sequence shown in SEQ ID NO: 12; and,
   (4) a nucleic acid able to function as a transcription regulatory region of torenia F3'5'H gene and having at least 90% sequence identity with the base sequence shown in SEQ ID NO: 12.
[9] An expression vector containing the nucleic acid described in [8] above.
[10] The expression vector described in [9] above, containing the base sequence shown in SEQ ID NO: 12.
[11] A non-human host transformed by the expression vector described in [9] or [10] above.
[12] A plant, or a progeny, a portion or a tissue thereof, introduced with the nucleic acid described in [8] above.
[13] The plant, or progeny, portion or tissue thereof, described in [12] above, which is a cut flower.
[14] A processed cut flower obtained by using the cut flower described in [13] above as a raw material.
[15] A nucleic acid selected from the group consisting of the following:
   (1) a nucleic acid composed of the base sequence shown in SEQ ID NO: 21;
   (2) a nucleic acid able to function as a transcription regulatory region of torenia F3'5'H gene and composed of a base sequence that has been modified by addition, deletion and/or substitution of one or several base sequences in the base sequence shown in SEQ ID NO: 21;
   (3) a nucleic acid able to function as a transcription regulatory region of torenia FNS gene and hybridize under highly stringent conditions with a nucleic acid composed of a base sequence complementary to the base sequence shown in SEQ ID NO: 21; and,
   (4) a nucleic acid able to function as a transcription regulatory region of torenia FNS gene and having at least 90% sequence identity with the base sequence shown in SEQ ID NO: 21.
[16] An expression vector containing the nucleic acid described in [15] above.
[17] The expression vector described in [16] above, containing the base sequence shown in SEQ ID NO: 21.
[18] A non-human host transformed by the expression vector described in [16] or [17] above.
[19] A plant, or a progeny, a portion or a tissue thereof, introduced with the nucleic acid described in [15] above.
[20] The plant, or progeny, portion or tissue thereof, described in [19] above, which is a cut flower.
[21] A processed cut flower obtained by using the cut flower described in [20] above as a raw material.
[22] An expression vector containing the nucleic acid described in [22] above and the nucleic acid described in [15] above.
[23] The expression vector described in [22] above, containing the base sequence shown in SEQ ID NO: 20 and the base sequence shown in SEQ ID NO: 21.
[24] A non-human host transformed by the expression vector described in [22] or [23] above.
[25] A plant, or a progeny, a portion or a tissue thereof, introduced with the nucleic acid described in [1] above or the nucleic acid described in [15] above.
[26] The plant, or progeny, portion or tissue thereof, described in [25] above, which is a cut flower.
[27] An expression vector containing the nucleic acid described in [8] above and the nucleic acid described in [15] above.
[28] The expression vector described in [27] above, containing the base sequence shown in SEQ ID NO: 12 and the base sequence shown in SEQ ID NO: 21.
[29] A non-human host transformed by the expression vector described in [27] or [28] above.
[30] A plant, or a progeny, a portion or a tissue thereof, introduced with the nucleic acid described in [8] above or the nucleic acid described in [15] above.
[31] The plant, or progeny, portion or tissue thereof, described in [30] above, which is a cut flower.
[32] A processed cut flower obtained by using the cut flower described in [31] above as a raw material.

### Effects of the Invention

The use of promoter regions thought to govern the transcription of enzyme genes in torenia flower petals, namely regions that control the transcription of torenia F3'5'H gene and FNS gene, makes it possible to specifically induce transcription of an exogenous gene in tissue such as flower tissue in which flavonoids and anthocyanins accumulate. Examples of transcribed exogenous genes include, but are not limited to, genes involved in flower color, scent and the like.

### BRIEF DESCRIPTION OF THE DRAWINGS

FIG. 1 is a schematic diagram of the biosynthesis pathway of flavonoids.
FIG. 2 is a schematic diagram of a binary vector pSPB3797 for gene insertion.
FIG. 3 is a schematic diagram of a binary vector pSPB3798 for gene insertion.

### BEST MODE FOR CARRYING OUT THE INVENTION

Examples of transcription regulatory regions of the present invention include nucleic acids composed of the base sequences shown in SEQ ID NO: 20, SEQ ID NO: 12 and SEQ ID NO: 21. However, promoters composed of base sequences that have been modified by addition, deletion and/or substitution of one or more (1, 2, 3, 4, 5, 6, 7, 8, 9 or 10) bases in the base sequence shown in SEQ ID NO: 20, SEQ ID NO: 12 or SEQ ID NO: 21 are also thought to maintain activity that is the same as that of the original promoter. Thus, the present invention also relates to a nucleic acid composed of a base sequence that has been modified by addition, deletion and/or substitution of one or several base sequences in the base sequence shown in SEQ ID NO: 20, SEQ ID NO: 12 or SEQ ID NO: 21 provided the nucleic acid functions as a transcription regulatory region in flower petals.

The present invention also relates to a nucleic acid that is able to function as a transcription regulatory region of torenia F3'5'H gene or FNS gene and hybridize under highly stringent conditions with the base sequence shown in SEQ ID NO: 20, SEQ ID NO: 12 or SEQ ID NO: 21, as well as a nucleic acid that is able to function as a transcription regulatory region of torenia F3'5'H gene or FNS gene and has at least 90% sequence identity with the base sequence shown in SEQ ID NO: 20, SEQ ID NO: 12 or SEQ ID NO: 21.

Examples of these nucleic acids include nucleic acids composed of a base sequence capable of hybridizing under stringent conditions with a polynucleotide complementary to the base sequence shown in SEQ ID NO: 20, SEQ ID NO: 12 or SEQ ID NO: 21 and having sequence identity with the base sequence shown in SEQ ID NO: 20, SEQ ID NO: 12 or SEQ ID NO: 21 of preferably about 70% or more, more preferably about 80%, 81%, 82%, 83%, 84%, 85%, 86%, 87%, 88%, 89%, 90%, 91%, 92%, 93%, 94%, 95%, 96%, 97% or 98%, and most preferably about 99%.

Here, the term "stringent conditions" refers to hybridization conditions easily determined by a person with ordinary skill in the art, and are typically experimental conditions dependent upon probe length, washing temperature and salt concentration. In general, the temperature for proper annealing becomes higher the longer the probe, while the temperature becomes lower the shorter the probe. Hybridization is generally dependent on the ability of a complementary strand to reanneal denatured DNA in the case of being present in an environment at a temperature close to but lower than the melting point thereof. More specifically, an example of lowly stringent conditions consists of washing in a 5×SSC and 0.1% SDS solution under temperature conditions of 37°C to 42°C in the stage of washing the filter following hybridization. In addition, an example of highly stringent conditions consists of washing in a 0.1×SSC and 0.1% SDS solution at 65°C. Increasing the stringency of these conditions allows the obtaining of a polynucleotide having higher sequence homology or sequence identity.

The present invention also relates to a vector containing the transcription regulatory region of torenia F3'5'H gene and/or FNS gene, and a non-human host transformed by that vector.

Moreover, the present invention also relates to a plant, or a progeny, portion or tissue thereof, having a useful trait such as change of color obtained by linking the transcription regulatory region of torenia F3'5'H gene and/or FNS gene to a useful exogenous gene and introducing that exogenous gene therein, and the portion may be a cut flower. Examples of plants able to be transformed include, but are not limited to, rose, chrysanthemum, carnation, snapdragon, cyclamen, orchid, dahlia, bluebell, freesia, Transvaal daisy, gladiola, baby's breath, kalanchoe, lily, pelargonium, geranium, petunia, torenia, tulip, rice, morning glory, barley, wheat, rape, potato, tomato, poplar, banana, eucalyptus, sweet potato, soybean, alfalfa, lupine and corn.

The present invention also relates to a processed product using the aforementioned cut flower (processed cut flower). Here, examples of processed cut flowers include, but are not limited to, pressed flowers, preserved flowers, dry flowers and resin-sealed flowers using these cut flowers.

### Examples

The following provides a detailed explanation of the invention according to examples thereof.

Molecular biological techniques were in accordance with Molecular Cloning (Sambrook and Russell, 2001) unless specifically indicated otherwise. Although methods used to transform Agrobacterium, petunia and rose are described in International Publication No. WO2004/020637 or Patent Document 4, the methods are not limited thereto.

### [Example 1: Cloning of Transcription Control Region of Torenia F3'5'H Gene TBG10]

The base sequence of torenia F3'5'H cDNA is known (Molecular Breeding 6, 239-246 (2000), Gene Bank DNA Accession No. AB012925). A chromosomal DNA library of torenia was prepared using λDASHII (Agilent Technologies, Inc.) for the vector in accordance with the method recommended by the manufacturer. Torenia chromosomal DNA was prepared from the leaves of the torenia variety Summer Wave Blue (Suntory Flowers Ltd.). The resulting torenia chromosomal DNA library was screened using labeled torenia F3'5'H cDNA and the plaque of a phage that hybridized with torenia F3'5'H cDNA was recovered. Using this phage as a template, PCR was carried out using two types of oligonucleotides (T3pro: 5'-AATTAACCCTCACTAAAGGG-3' (SEQ ID NO: 1), T7pro: 5'-TAATACGACTCACTATAGGG-3' (SEQ ID NO: 2)) as primers to amplify a DNA fragment containing the sequence derived from torenia chromosomal DNA. Using this DNA as a template, PCR was carried out using one set of oligonucleotides (T3pro, THF2RV: 5'-CTATGGAAGATAACAATG-3' (SEQ ID NO: 3)) or one set of oligonucleotides (T7pro, THF2RV) as primers. An approximately 5.6 kb fragment (SEQ ID NO: 4) able to be amplified by PCR using T3pro and THF2RV was cloned in pCR2.1 TOPO (Invitrogen Corp.). The resulting plasmid was designated as pSPB3745.

Using the aforementioned phage as a template, PCR was carried out using one set of oligonucleotides (TBG10proFW: 5'-TGAAATATAAATATGAATGGG-3' (SEQ ID NO: 5), TBG10proRV: 5'-ACTGAATGGTGACTAGCTGC-3' (SEQ ID NO: 6)) as primers. The resulting DNA fragment was cloned in BluntII-TOPO (Invitrogen Corp.) to obtain plasmid pSPB3764 (containing SEQ ID NO: 7). This DNA sequence is shown in SEQ ID NO: 7. Moreover, using pSPB3764 as a template, PCR was carried out using one set of oligonucleotides (TBG10proFW, TBG10proXbaIRV: 5'-TCTAGACTGAATGGTGACTAGC-3' (SEQ ID NO: 8)) as primers. This DNA fragment was cloned in BluntII-TOPO to obtain plasmid pSPB3770. This plasmid contains in an approximately 4kb 5'-untranslated region of torenia F3'5'H and has a restrictase XbaI recognition sequence on the 3'-terminal thereof.

### [Example 2: Cloning of Transcription Control Region of Torenia F3'5'H Gene TBG16]

Plaque of the phage that hybridized with torenia F3'5'H cDNA obtained in Example 1 was allowed to proliferate to obtain phage DNA. Using this as a template, PCR was carried out using one set of oligonucleotides (T3pro, THF2RV) or one set of oligonucleotides (T7pro, THF2RV) as primers. An approximately 2.3 kb DNA fragment obtained from the (T3pro, THF2RV)-derived PCR product was cloned in pCR2.1 TOPO to obtain plasmid pSPB3746. The sequence thereof is shown in SEQ ID NO: 9. Using this plasmid as a template, PCR was carried out using a set of oligonucleotides (TBG16proFW: 5'-TCCTATTGCACTCGTTTTTTC-3' (SEQ ID NO: 10), TBG16proRV: 5'-ACTGAATGGTGACTAGCCGC^3' (SEQ ID NO: 11)) as primers. The resulting DNA fragment was cloned in BluntII-TOPO (Invitrogen Corp.) to obtain plasmid pSPB3758. The DNA sequence contained in this plasmid is shown in SEQ ID NO: 12. Moreover, using pSPB3758 as a template, PCR was carried out using one set of oligonucleotides (TBG16proFW, TBG16proBamHI: 5'-GGATCCACTGAATGGTGACTAGCC-3' (SEQ ID NO: 13)) as primers. This DNA fragment was cloned in BluntII-TOPO to obtain plasmid pSPB3768. This plasmid contains in an approximately 0.7 kb 5'-untranslated region of torenia F3'5'H and has a restrictase BamHI recognition sequence on the 3'-terminal thereof.

### [Example 3: Cloning of Transcription Control Region of Torenia FNS Gene]

The torenia chromosomal DNA library obtained in Example 1 was screened using labeled torenia FNS cDNA, and plaque of a phage hybridized with torenia FNS cDNA was recovered. The phage plaque was allowed to proliferate to prepare phage DNA. Using this DNA as a template, PCR was carried out using one set of oligonucleotides (T3pro, TFNSR3: 5'-ATTCCTAATGGGCTGAAAGTG-3' (SEQ ID NO: 14)) or one set of oligonucleotides (T7pro, TFNSR3) as primers. An approximately 4.2 kb DNA fragment (SEQ ID NO: 15) able to be amplified by PCR using T7pro and TFNSR3 was cloned in pCR2.1 TOPO. The resulting plasmid was designated as pSPB3747.

Using the aforementioned phage DNA as a template, PCR was carried out using one set of oligonucleotides (TFNS1proFW: 5'-CAAATGAAACCCCATCAGTGTC-3' (SEQ ID NO: 16), TFNS1proRV: 5'-GCTTTATATATATTTTTTTAGCGC-3' (SEQ ID NO: 17)) as primers. The amplified DNA fragment was cloned in BluntII-TOPO to obtain plasmid PSPB3759. The sequence inserted with this plasmid is shown in SEQ ID NO: 18. Using pSPB3759 as a template, PCR was carried out using one set of oligonucleotides (TNFS1proFW, TFNS1pBamHIRV: 5'-GGATCCGCTTTATATATATTTTTTTAGC-3' (SEQ ID NO: 19) as primers. This DNA fragment was cloned in BluntTOPO to obtain plasmid pSPB3769. This plasmid contains an approximately 3.6 kb 5'-untranslated region of torenia FNS and has a restrictase BamHI recognition sequence on the 3'-terminal thereof.

### [Example 4: Production of Binary Vector Containing Torenia F3'5'H and FNS Transcription Control Regions]

The plasmid pSPB3770 obtained in Example 1 was digested and blunted with SspI and further digested with XbaI. The resulting 1.6 kb DNA fragment (SEQ ID NO: 20) was linked to a DNA fragment obtained by digesting and blunting pBinPLUS with HindIII and further digesting with XbaI to obtain plasmid pSPB3791.

A DNA fragment obtained by digesting plasmid pSPB580 (described in Patent Document 4) with BamHI and PacI (containing pansy-derived F3'H'5H BP40 cDNA and a petunia D8 terminator sequence) was linked to pBinPLUS obtained by digesting with BamHI and PacI to obtain plasmid pSPB3795. A DNA fragment obtained by digesting plasmid pSPB3791 with XbaI and PacI was linked to a DNA fragment obtained by digesting pSPB3795 with XbaI and PacI to obtain binary vector pSPB3793. A TBG10-derived transcription control region, pansy-derived F3'5'H cDNA and the petunia D8 terminator were linked in this binary vector in that order.

The plasmid pSPB3768 obtained in Example 2 was digested and blunted with EcoRI and further digested with BamHI. The resulting approximately 0.7 kb DNA fragment was linked to a DNA fragment obtained by digesting and blunting pBinPLUS with HindIII and further digesting with BamHI to obtain plasmid pSPB3790. A DNA fragment obtained by digesting this with BamHI and PacI was then linked to a DNA fragment obtained by digesting pSPB580 with BamHI and PacI to obtain binary vector pSPB3792. A TBG16-derived transcription control sequence, pansy-derived F3'5'H cDNA and the petunia D8 terminator were linked in this binary vector in that order.

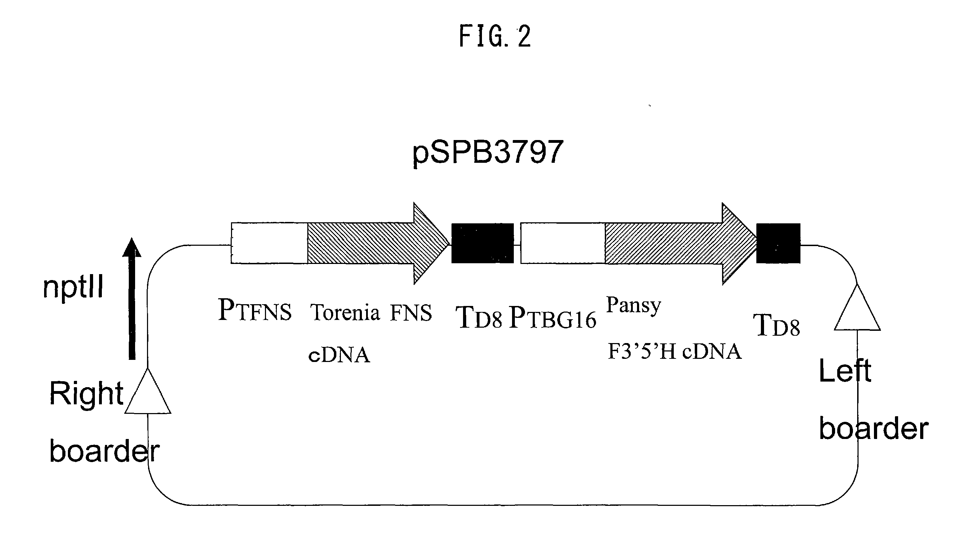

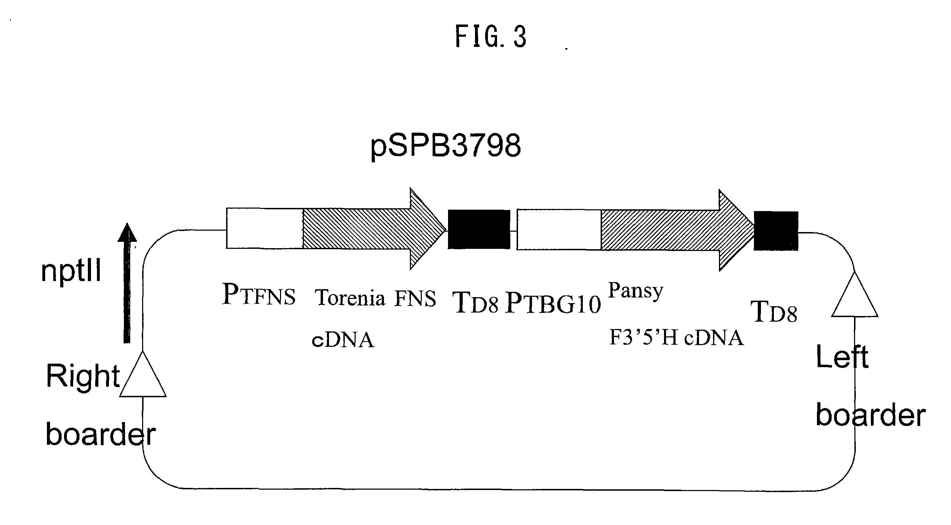

The plasmid pSPB3769 obtained in Example 3 was digested and blunted with SpeI and further digested with BamHI. The resulting approximately 1.4 kb DNA fragment (SEQ ID NO: 21) was linked to a DNA fragment obtained by digesting and blunting pSPB3383 with HindIII and further digesting with BamHI to obtain plasmid pSPB3789. A torenia FNS transcription control region, torenia FNS cDNA and petunia D8 terminator were linked in this plasmid in that order, and a DNA fragment in which they were contained was able to be recovered by cleaving with AscI. The binary vector introduced with this DNA fragment at the AscI site of pSPB3793 was designated as pSPB3798 (see FIG. 3), while the binary vector introduced with this DNA fragment at the AscI site of pSPB3792 was designated as pSPB3797 (see FIG. 2).

### [Example 5: Expression in Petunia (Results for PT315)]

Plasmid pSPB3797 or pSPB3798 described in Example 4 was introduced into Agrobacterium tumefaciens. Petunia strain Skr4xSw63 (described in International Publication No. WO93/01290) that produces light pink-colored flowers was transformed using this transformed Agrobacterium. An experimental plot derived from pSPB3797 was designated as PT314 and an experimental plot derived from pSPB3798 was designated at PT315. Two independent strains of transformants were obtained in PT314. The flower color of both strains changed to light reddish-violet. The results of analyzing the flower petals of PT314 and PT315 and the anthocyanidins of Skr4xSw63 using known methods are shown in the following Tables 1 and 2.

**[Table 1]**

| Sample | Delphinidin | Petunidin | Pelargonidin | Peonidin | Malvidin |
|---|---|---|---|---|---|
| PT314-1 | 5.4 | 98.6 | 3.8 | 20.6 | 1950 |
| PT314-2 | 4.2 | 76.5 | 3.3 | 17.6 | 1150 |
| Skr4xSw63 | 0 | 2.9 | 22.5 | 15.8 | 60.3 |

Units: µg/g fresh flower petals

Twenty-six independent strains of transformants were obtained in PT315. Among these, the flower color of 15 strains changed to reddish-violet. The flower petals of a portion of these strains and the anthocyanidins and flavones of Skr4xSw63 were analyzed using known methods.

**[Table 2]**

| Sample | Delphinidin | Petunidin | Pelargonidin | Peonidin | Malvidin | Apigenin | Tricetin |
|---|---|---|---|---|---|---|---|
| PT315-1 | 3.0 | 72 | 0 | 2.7 | 1330 | 2.8 | 11 |
| PT315-9 | 6.8 | 126 | 0 | 0.6 | 1580 | 1.9 | 31 |
| PT315-12 | 2.0 | 33 | 1.0 | 7.0 | 393 | 44 | 9.4 |
| PT315-13 | 3.0 | 72 | 0 | 2.8 | 1550 | 4.9 | 17 |
| PT315-14 | 4.0 | 72 | 0 | 5.3 | 1290 | 5.6 | 11 |
| PT315-15 | 5.0 | 109 | 0 | 1.0 | 1630 | 4.6 | 30 |
| PT315-17 | 5.6 | 116 | 0 | 8.2 | 1690 | 18 | 27 |
| PT315-19 | 1.9 | 39 | 1.5 | 11 | 756 | 3.0 | 7.0 |
| PT315-20 | 3.6 | 93 | 0 | 4.8 | 1190 | 3.3 | 17 |
| PT315-21 | 10 | 185 | 0 | 14 | 1130 | 1.4 | 8.1 |
| PT315-25 | 5.8 | 101 | 0 | 0.6 | 1540 | 2.4 | 11 |
| PT315-26 | 3.3 | 71 | 0 | 1 | 862 | 3.5 | 25 |
| Skr4xSw63 | 0 | 3.2 | 5.6 | 11 | 74 | 0 | 0 |

Units: µg/g fresh flower petals

The above results indicate that a TBG10-derived transcription control region contained in pSPB3770, a TBG16-derived transcription control region contained in pSPB3768, and an FNS-derived transcription control region contained in pSPB3759 demonstrate their functions in different strains of petunia as well.

### [Example 6: Introduction of pSPB3798 into Rose Variety "Ocean Song"]

Binary plasmid pSPB3798 described in Example 4 was introduced into Agrobacterium tumefaciens strain Agl0. Rose variety "Ocean Song" was transformed using this transformed Agrobacterium to obtain 15 transformants. Among the 15 transformants subjected to anthocyanidin analysis, accumulation of delphinidin was confirmed in 5 transformants. The maximum delphinidin content was 7.3% (mean: 5.0%). In addition, as a result of analyzing flavonols and flavones, myricetin was detected in 8 of 9 transformants and tricetin was detected in 7 transformants. Delphinidin, myricetin and tricetin were all formed by the action of pansy-derived F3'5'H, and the transcription control region of torenia-derived TBG10 gene was indicated to function in rose.

On the other hand, since previously absent accumulation of flavones (tricetin, luteolin, apigenin) was able to be confirmed in all the transformants subjected to analysis, the transcription control region of torenia-derived FNS gene was also indicated to function in rose. Furthermore, the total amount of flavones was a maximum of 0.6588 mg per 1 g of fresh flower petals.

The analysis values of representative transformants are shown in the following Table 3.

**[Table 3]**

| Plant No. | Del (%) | Anthocyanidins (mg/g) | | | Flavonols (mg/g) | | | Flavones (mg/g) | | | |
|---|---|---|---|---|---|---|---|---|---|---|---|
| | | Del | Cya | Pel | M | Q | K | Tri | Lut | Api | Total |
| Ocean Song Control | 0.05 | 0.0000 | 0.0324 | 0.0000 | 0.0000 | 2.6657 | 0.1730 | 0.0000 | 0.0000 | 0.0000 | 0.0000 |
| 1 | 7.3% | 0.0006 | 0.0076 | 0.0000 | | | | | | | |
| 2 | 6.0% | 0.0006 | 0.0100 | 0.0000 | 0.0662 | 1.3835 | 0.2738 | 0.0431 | 0.2928 | 0.0138 | 0.3498 |
| 3 | 5.4% | 0.0002 | 0.0036 | 0.0000 | | | | | | | |
| 4 | 4.8% | 0.0004 | 0.0090 | 0.0000 | | | | | | | |
| 5 | 1.4% | 0.0002 | 0.0141 | 0.0000 | 0.0109 | 1.7564 | 0.2911 | 0.0110 | 0.1680 | 0.0088 | 0.1878 |
| 6 | 0.0% | 0.0000 | 0.0019 | 0.0000 | 0.0041 | 0.5825 | 0.2806 | 0.0112 | 0.3098 | 0.0206 | 0.3417 |
| 7 | 0.0% | 0.0000 | 0.0026 | 0.0000 | 0.0000 | 0.05060 | 0.3112 | 0.0063 | 0.3153 | 0.0222 | 0.3439 |
| 8 | 0.0% | 0.0000 | 0.0106 | 0.0000 | 0.0075 | 1.4460 | 0.4400 | 0.0056 | 0.0825 | 0.0059 | 0.0941 |
| 9 | 0.0% | 0.0000 | 0.0037 | 0.0000 | 0.0137 | 0.8253 | 0.2856 | 0.0577 | 0.5679 | 0.332 | 0.6588 |
| 10 | 0.0% | 0.0000 | 0.0492 | 0.0000 | 0.0341 | 3.5195 | 0.4933 | 0.0000 | 0.1222 | 0.0066 | 0.1289 |

| | | | | | | | | | | | |
|---|---|---|---|---|---|---|---|---|---|---|---|
| Del: delphinidin, Cya: cyanidin, Pel: pelargonidin, M: myricetin, Q: quercetin, K: kaempferol, Tri: tricetin, Lut: luteolin, Api: apigenin Del(%): Percentage of delphinidin among total anthocyanidins | | | | | | | | | | | |

### [Example 7: Introduction of pSPB3797 into Rose Variety "Ocean Song"]

Binary vector pSPB3797 described in Example 4 was introduced into the lavender-colored rose variety "Ocean Song" and 7 transformants were obtained. Accumulation of delphinidin was unable to be confirmed in any of the 7 transformants analyzed for anthocyanidins. In addition, as a result of analyzing for flavonols and flavones, there were no transformants in which myricetin or tricetin was detected. These results indicate that the promoter of torenia-derived TBG16 gene does not function in rose.

On the other hand, since accumulation of flavones (luteolin, apigenin) due to the action of torenia-derived FNS was able to be confirmed in all analyzed transformants, the promoter of torenia-derived FNS gene was indicated to function in rose. Furthermore, the total amount of flavones was a maximum of 0.1704 mg per 1 g of fresh flower petals.

Analysis values of representative transformants are shown in the following Table 4.

**[Table 4]**

| Plant No. | Del (%) | Anthocyanidins (mg/g) | | | Flavonols (mg/g) | | | Flavones (mg/g) | | | |
|---|---|---|---|---|---|---|---|---|---|---|---|
| | | Del | Cya | Pel | M | Q | K | Tri | Lut | Api | Total |
| Ocean Song Control | 0.05 | 0.0000 | 0.0324 | 0.0000 | 0.0000 | 2.6657 | 0.1730 | 0.0000 | 0.0000 | 0.0000 | 0.0000 |
| 1 | 0.0% | 0.0000 | 0.0123 | 0.0000 | 0.0000 | 0.1610 | 0.2288 | 0.0000 | 0.1405 | 0.0076 | 0.1481 |
| 2 | 0.0% | 0.0000 | 0.0034 | 0.0000 | 0.0000 | 0.8202 | 0.2264 | 0.0000 | 0.1367 | 0.0082 | 0.1449 |
| 3 | 0.0% | 0.0000 | 0.0106 | 0.0000 | 0.0000 | 1.2625 | 0.5670 | 0.0000 | 0.1292 | 0.0102 | 0.1394 |
| 4 | 0.0% | 0.0000 | 0.0190 | 0.0000 | 0.0000 | 1.9873 | 0.9176 | 0.0000 | 0.0702 | 0.0048 | 0.0750 |
| 5 | 0.0% | 0.0000 | 0.0077 | 0.0000 | 0.0000 | 1.6323 | 0.4398 | 0.0000 | 0.1651 | 0.0053 | 0.1704 |
| 6 | 0.0% | 0.0000 | 0.0208 | 0.0000 | 0.0000 | 2.4281 | 0.6282 | 0.0000 | 0.1067 | 0.0052 | 0.1119 |
| 7 | 0.0% | 0.0000 | 0.0154 | 0.0000 | 0.0000 | 2.1013 | 0.3322 | 0.0000 | 0.1528 | 0.0062 | 0.1590 |

| | | | | | | | | | | | |
|---|---|---|---|---|---|---|---|---|---|---|---|
| Del: delphinidin, Cya: cyanidin, Pel: pelargonidin, M: myricetin, Q: quercetin, K: kaempferol, Tri: tricetin, Lut: luteolin, Api: apigenin Del(%): Percentage of delphinidin among total anthocyanidins | | | | | | | | | | | |

### INDUSTRIAL APPLICABILITY

Promoter regions thought to govern transcription of enzyme genes in torenia flowers, namely the transcription regulatory regions of torenia flavonoid 3',5'-hydroxlyase gene and flavone synthase gene, were determined to function as transcription regulatory regions in the flower petals of different species of flowers in the form of petunia and rose. Thus, it is possible to specifically induce transcription of an exogenous gene in tissue such as flower tissue in which anthocyanins accumulate by using these transcription regulatory regions. Examples of transcribed exogenous genes include, but are not limited to, genes involved in flower color, scent and the like.

## Claims

1. A nucleic acid selected from the group consisting of the following:
(1) a nucleic acid composed of the base sequence shown in SEQ ID NO: 20;
(2) a nucleic acid able to function as a transcription regulatory region of torenia F3'5'H gene and composed of a base sequence that has been modified by addition, deletion and/or substitution of one or several base sequences in the base sequence shown in SEQ ID NO: 20;
(3) a nucleic acid able to function as a transcription regulatory region of torenia F3'5'H gene and hybridize under highly stringent conditions with a nucleic acid composed of a base sequence complementary to the base sequence shown in SEQ ID NO: 20; and,
(4) a nucleic acid able to function as a transcription regulatory region of torenia F3'5'H gene and having at least 90% sequence identity with the base sequence shown in SEQ ID NO: 20.

2. An expression vector containing the nucleic acid according to claim 1.

3. The expression vector according to claim 2, containing the base sequence shown in SEQ ID NO: 20.

4. A non-human host transformed by the expression vector according to claim 2 or 3.

5. A plant, or a progeny, a portion or a tissue thereof, introduced with the nucleic acid according to claim 1.

6. The plant, or progeny, portion or tissue thereof, according to claim 5, which is a cut flower.

7. A processed cut flower obtained by using the cut flower according to claim 6 as a raw material.

8. A nucleic acid selected from the group consisting of the following:
(1) a nucleic acid composed of the base sequence shown in SEQ ID NO: 12;
(2) a nucleic acid able to function as a transcription regulatory region of torenia F3'5'H gene and composed of a base sequence that has been modified by addition, deletion and/or substitution of one or several base sequences in the base sequence shown in SEQ ID NO: 12;
(3) a nucleic acid able to function as a transcription regulatory region of torenia F3'5'H gene and hybridize under highly stringent conditions with a nucleic acid composed of a base sequence complementary to the base sequence shown in SEQ ID NO: 12; and,
(4) a nucleic acid able to function as a transcription regulatory region of torenia F3'5'H gene and having at least 90% sequence identity with the base sequence shown in SEQ ID NO: 12.

9. An expression vector containing the nucleic acid according to claim 8.

10. The expression vector according to claim 9, containing the base sequence shown in SEQ ID NO: 12.

11. A non-human host transformed by the expression vector according to claim 9 or 10.

12. A plant, or a progeny, a portion or a tissue thereof, introduced with the nucleic acid according to claim 8.

13. The plant, or progeny, portion or tissue thereof, according to claim 12, which is a cut flower.

14. A processed cut flower obtained by using the cut flower according to claim 13 as a raw material.

15. A nucleic acid selected from the group consisting of the following:
(1) a nucleic acid composed of the base sequence shown in SEQ ID NO: 21;
(2) a nucleic acid able to function as a transcription regulatory region of torenia F3'5'H gene and composed of a base sequence that has been modified by addition, deletion and/or substitution of one or several base sequences in the base sequence shown in SEQ ID NO: 21;
(3) a nucleic acid able to function as a transcription regulatory region of torenia FNS gene and hybridize under highly stringent conditions with a nucleic acid composed of a base sequence complementary to the base sequence shown in SEQ ID NO: 21; and,
(4) a nucleic acid able to function as a transcription regulatory region of torenia FNS gene and having at least 90% sequence identity with the base sequence shown in SEQ ID NO: 21.

16. An expression vector containing the nucleic acid according to claim 15.

17. The expression vector according to claim 16, containing the base sequence shown in SEQ ID NO: 21.

18. A non-human host transformed by the expression vector according to claim 16 or 17.

19. A plant, or a progeny, a portion or a tissue thereof, introduced with the nucleic acid according to claim 15.

20. The plant, or progeny, portion or tissue thereof, according to claim 19, which is a cut flower.

21. A processed cut flower obtained by using the cut flower according to claim 19 as a raw material.

22. An expression vector containing the nucleic acid according to claim 1 and the nucleic acid according to claim 15.

23. The expression vector according to claim 22, containing the base sequence shown in SEQ ID NO: 20 and the base sequence shown in SEQ ID NO: 21.

24. A non-human host transformed by the expression vector according to claim 22 or 23.

25. A plant, or a progeny, a portion or a tissue thereof, introduced with the nucleic acid according to claim 1 or the nucleic acid according to claim 15.

26. The plant, or progeny, portion or tissue thereof, according to claim 25, which is a cut flower.

27. An expression vector containing the nucleic acid according to claim 8 and the nucleic acid according to claim 15.

28. The expression vector according to claim 27, containing the base sequence shown in SEQ ID NO: 12 and the base sequence shown in SEQ ID NO: 21.

29. A non-human host transformed by the expression vector according to claim 27 or 28.

30. A plant, or a progeny, a portion or a tissue thereof, introduced with the nucleic acid according to claim 8 or the nucleic acid according to claim 15.

31. The plant, or progeny, portion or tissue thereof, according to claim 30, which is a cut flower.

32. A processed cut flower obtained by using the cut flower according to claim 31 as a raw material.


SEQUENCE LISTING

<110>  Suntory Holdings Limited

<120>  Promoters derived from Trenia Functioning in Petals

<130>  SP2012-0137(AB549)

<150>  JP2012-038644
<151>  2012-02-24

<160>  21

<170>  PatentIn version 3.5

<210>  1
<211>  20
<212>  DNA
<213>  Artificial Sequence

<220>
<223>  primer T3pro

<400>  1
aattaaccct cactaaaggg                                                    20


<210>  2
<211>  20
<212>  DNA
<213>  Artificial Sequence

<220>
<223>  primer T7pro

<400>  2
taatacgact cactataggg                                                    20


<210>  3
<211>  18
<212>  DNA
<213>  Artificial Sequence

<220>
<223>  primer THF2RV

<400>  3
ctatggaaga taacaatg                                                      18


<210>  4
<211>  5606
<212>  DNA
<213>  Torenia Fournieri


<220>
<221>  misc_feature
<223>  Trenia F3'5'H TBG10 region in pSPB3745

<400>  4
aattaaccct cactaaaggg aacgaattcg gatcctcatg ttatgaaata taaatatgaa        60

tgggattaaa cttgtagtta attggtatca ttaaaaaaaa aagaaaaaag aaattgaagt 120

aaaataatcg caggtaactt gaagaaaata ttaataacta aaataataat aataataata 180

ataataataa gcaggtttga aacacacacc ttgggaattt gttgttcttg acagcctttt 240

gaccatattt cctccatcga tatccatcgt caagtatatc gacttggctt cttgtctgaa 300

aggcgtattt atgcttctta ttcttcttct tcttattctt ctcctcgctc tttattgctt 360

ccttattatt atcagacttg tttgggtcct cctctgcgag agttattgca gtactattat 420

tattaaagct actactggcg attgtaatag catctgacat gcttttaata ttcatctcca 480

tgctgttata gccaaaacta ctactgctag gcctgtgatc ttgaaacaga tcataatgat 540

cttgtggaaa tgaagaagaa gactaaaaca ttattggata gttattattg tccatgagaa 600

gagaagatga taatagataa caaataaatg atggttttcc ttgttttttt ttataataac 660

tatgaatgtg aagaatagtg gattaagaaa agtacaagtc gtcgtgtggt gttgtttaga 720

atgagagaaa agtttattat acaacaagta ccgtcccagc tcgtttaaag atgaaaggcg 780

gcggcttgac ttgagggcga atattattat ttatttaaat aagttacatc ataaacatat 840

tttgtgcata gtgaatgtta aaccaaaagc gaaatggcta tgatcgcagt attaatatat 900

tatctgagga gataaatttg atcgaattga ttagcaggga taataattat agagatgatc 960

caacttgctt gatgaaatta tggatctata ctcaatctta aaacgaattt acttacttac 1020

atagcgtgaa atagctaatt ggaaatgaaa agaaaaagaa aaagaaaaat tgttagaaat 1080

atgacttgga gggagggttt tgaatcttca aacatacaca aatttaatta atacattacg 1140

gtcgttaagc aaatcatttt tattctacgg acagtagata ttgcaataat agttagaaaa 1200

gttggtaaag aattagtaca gttggtcaaa cactattatt attattatta ttattgttat 1260

tattattatt attctataaa tctgataata aaaacaaata gcatatgaat aatagcatat 1320

aaatgctgga tccgatttaa ttttaatttt agttaaatag caactttcag taaatttttt 1380

tagttaactg cctctggaaa aatgaaatgc gtgggtggga gcggttcgga tttggtacgg 1440

aaaatgcttt tctccgcatt gtgcggataa atctggaccg tgcgatcagg cccaaaatta 1500

atccagtggt gaaggacgag agggggtaaa tctggaattt ttttttttac tcataataaa 1560

aaacgattat atcttttgaa tgccaaaaca aaataaaaaa ataaaaacac caaatttcat 1620

catttttaat tccctacaaa aagagatcca tcttgctata taaattttat attttagggg 1680

tttagggttt agggtttacg gcctggcaca gacacatcta aaacgaagca caactcagct 1740

aaacgaagca caaattcatt attatgaagc acatattcat tagtatgaag cacatattca 1800

tttttatgaa gcgcaattca tttgtatgaa gcacatattc atttttatga agcgcaattc 1860

atttgtatga agcacaaaat catttgtatg aaacttataa tcatttgtat gaagcacaaa 1920

ttcatttata tgaagcaaaa attcatttgt atgaacgcaa ttcatttgta tgaatcacaa 1980

attaatttgt atgaagcaca aattaatttg tatgaagcac aactcatttg tacaaaatca 2040

tacaaataat tataagtacc atctaattga agcacaaatt catttgtttg tatgaagcac 2100

aaattcattt gtataaagca caaattcatt tgtacaatac atccaaataa tatataaagc 2160

accatctaaa cgaagtacaa attcatttgt atgaagcaca aattcgtaca aaaaatattt 2220

aaagcacaat ctaaaacgaa gcacagattc atttgtatga agcacaaatt catttgtaca 2280

atacatccaa ataatatata aagcaccatc taaacgaagc acaaattcat ttgtatgaag 2340

cacaaattca tttgtacaaa ttcatacaaa aaatatttaa agcacaatct aaaacgaagc 2400

acagattcat ttgtatgaag cacagattca tcaggggtat tttagacaaa aacccgacga 2460

atatcatccg ttagatttat tttgagtaaa tccaatggcc cattactttt gtccgcacat 2520

acggagaaaa gtaccgtccg catttgaacc agaggggatg ggagggactg cgaacataat 2580

ataattcggg tcgatggcgt accaaaaaat ggtcaaataa ataatgctac aagttaaatg 2640

cacgtgattc tcttgttgac cgttaaatgt tgatttctat tttgatcgaa ttttatagat 2700

attagctcag ctgctgagac tgagagagag attattttag aaacggatct gatcaagatc 2760

gctcgtgatt attctcatca tctcgtgtag catttagatt tggtaaaacc gtaaaaatat 2820

cttgtcttaa cttcttccta ttaattccac aatctcacgc aaaaatgtat ttttacagcg 2880

acatgccttg aggaggaatt tcagtgctaa agacaaaata gacttagaaa tgtgccgttt 2940

gattaatttt ggtagttaaa aaaaaaaaat tatttattta tttatgctta gccataataa 3000

ttaggacgga aacaagaaga atttgcccat ttttaaggcc tacgaatagt atacgggcca 3060

taactttatg ggggacattt tcaggcctac ccaatatgat tgaatgtgcc tccactccaa 3120

ttatcaaaaa gaaacgccga ctgagcttga gcacgatttt caaatgtcta ctccacagtg 3180

caaaaattgc acgatcaatt ctgatgaaat gtattcccat ctatgtagat gaatgggagc 3240

gtttgggatc caacactcgt tttttctacc tatttcgacg gttttctctg gtcaggtggt 3300

ttccgagagg ggactgaaat atacagtaca gttgttgaga tattgatgag atcaatcaag 3360

tcgtgtaagc ttatctttct gaaaatataa ttatatttaa ttgattatcc taccgcttgt 3420

caattttttc tattgcgagt gtgttttggt gtaaagagta gctatcgatg ctgaagagat 3480

ttttatcaag taagatgatg tggagaacta actgattgtt tgttttgttg ccagcgatat 3540

attgatgatg atgttctttg caattagagt tattctactt aacaaacttt ttctcttgtc 3600

ataacataca catagatact gcatgctttt ccatgattca ttctatctaa taaaggtcaa 3660

tctactcccc actttcatcc atgccaatga aacacataca taaatggaaa catatcataa 3720

tttaggtgtg gccctattag ttttcttctc gttgaacgtc ggttgatggc ctaaaaaaac 3780

gtgacaaacc acacagttgt cacccaacgt actccattga tacacatata aagctcagtg 3840

attgattatt cataaacact caactggttg agcactattt gcctcttctt tgctagattc 3900

acatcgagta agtactccgt ccgacctttt attgtatggt ataatcgtaa gaaaacaaat 3960

taagcagcta gtcaccattc agtatgacag tagctactac acttgtcgtt gtgttgagta 4020

gagagttgtt gtatgtgttg gttgtctaca ttctggtctc aaaatcacta tctaccatca 4080

tcgtcgtctc ccgcaaacgg ttaccccccg gcccaacggg cttccccgtg gtcggtgccc 4140

tcccattgtt gggcagcatg cctcacgtgg cgttggccaa aatggcaaaa aaatacgggc 4200

ccgtcatgta ccttaaaacg ggcacactgg gcatggtggt ggcctccacg ccttcctcgg 4260

caagagcgtt tctcaagaca ctggactcca acttctcgaa ccggcccgcc aatgccggtg 4320

ccacccacct ggcctacggt gcgcaggaca tggtgttcgc tccctacggc cccagatgga 4380

gactgctgag aaggttaagc aacctccaca tgctcggcgg caaggccctc gacggatggg 4440

caaacgtcag ggcctcggag ttggggcaca tgctggaggc catgaaccgg gcgagccggg 4500

agggggaggt ggtggtggtg ccggagatgc tggtgtacgc catggccaac atgatcgggc 4560

aagtcacact gagccggcgg gtcttcgtga ccaaagggga ggaagtgaac gagtttaagg 4620

acatggtggt ggagctcatg acgtctgctg gatacttcaa catcggggac tttgtcccga 4680

tccttgcttg gatggacttg caagggattg agagagggat gaaagctctt cacaaaaagt 4740

ttgacaagct catcacgaga atgttggagc agcatgaggc gtcggcccac aagcccgatt 4800

ttctcgacgc catcatcgcg gccagccttg acgattattc gagtgaggag gagaggctca 4860

gtacgtccaa catcaaagct ctcttattgg taaggctaag ctttattaat tttacccatc 4920

attgaatatg catgcaacaa caagaggtcc tatcattgaa tatgcatgtg tgtgtgtaga 4980

acttgtttac tgctggaacg gacacatcat cgagcacgat agaatgggca ctctcggaga 5040

tgctgaaaaa ggggaaaatc ctaaagaggg ctcaagagga gatggacaga gtggtgggac 5100

gagagaggcg cttggtggaa tcggacatag agaaactggg gtacctgaaa gccatatgca 5160

aagagacgtt ccggaagcat ccttcgacgc cgctgaacct gcctcggatc tcgtcggagg 5220

catgcgtggt gaacggccac tacataccaa agggcacgag gctgagcgtc aacatatggg 5280

ccatcgggag agaccctgag gtgtgggagg atccgttgga gtttaacccg gacaggtttc 5340

tgcactcgaa gatggatccg agggggaacg atttcgagct catgcccttc ggtgcgggtc 5400

gaaggatatg tgccgggacg aggatgggga tagtcctcgt cgagtatata ttggccactt 5460

tggtgcattc tttcggctgg aaagcggctg atcaagataa caacatcatg aatatggagg 5520

aggcctttgg gattgctctg cagaaggcca cacctcttaa ggctctcgtt actcctcgcc 5580

tgcctttaca ttgttatctt ccatag 5606

<210> 5

<211>  21
<212>  DNA
<213>  Artificial Sequence

<220>
<223>  primer TBG10proFW

<400>  5
tgaaatataa atatgaatgg g                                                21


<210>  6
<211>  20
<212>  DNA
<213>  Artificial Sequence

<220>
<223>  primer TBG10proRV

<400>  6
actgaatggt gactagctgc                                                  20


<210>  7
<211>  3942
<212>  DNA
<213>  Torenia Fournieri


<220>
<221>  misc_feature
<223>  Trenia F3'5'H TBG10  in pSPB3764

<220>
<221>  primer_bind
<222>  (1)..(21)
<223>  primer TBG10proFW binding

<220>
<221>  primer_bind
<222>  (3923)..(3942)
<223>  primer TBGproRV binding

<400>  7
tgaaatataa atatgaatgg gattaaactt gtagttaatt ggtatcatta aaaaaaaaag      60

aaaaaagaaa ttgaagtaaa ataatcgtag gtaacttgaa gaaaataata ataactaaaa     120

taataataat aataataata ataataagca ggtttgaaac acacaccttg ggaatttatt     180

gttcttgaca gccttttgac catatttcct ccatcgatat ccatcgtcaa gtatatcgac     240

ttggcttctt gtctgaaagg cgtatttatg cttcttattc ttcttcttct tattcttctc     300

ctcgctcttt attgcttcct tattattatc agacttgttt gggtcctcct ctgcgagagt     360

tattgcagta ctattattat taaagctact actggcgatt gtaatagcat ctgacatgct     420

tttaatattc atctccatgc tgttatagcc aaaactacta ctgctaggct tgtgatcttg     480

aaacagatca taatgatctt gtggaaatga agaagaagac taaaacatta ttggatagtt     540

attattgtcc atgagaagag aagatgataa tagataacaa ataaatgatg gttttccttg     600

tttttttttt ataataacta tgaatgtgaa gaatagtgga ttaagaaaag tacaagtcgt 660

cgtgtggtgt tgtttagaat gagagaaaag tttattatac aacaagtacc gtcccagctc 720

gtttaaagat gaaaggcggc ggcttgactt gagggcgaat attattattt atttaaataa 780

gttacatcat aaacatattt ttgtgcatag tgaatgttaa accaaaagcg aaatggctat 840

gatcgcagta ttaatatatt atctgaggag ataaatttga tcgaattgat tagcagggat 900

aataattata gagatgatcc aacttgcttg atgaaattat ggatctatac tcaatcttaa 960

aacgaattta cttacttaca tagcgtgaaa tagctaattg gaaatgaaaa gaaaaagaaa 1020

aagaaaaatt gttagaaata tgacttggag ggagggtttt gaatcttcaa acatacacaa 1080

atttaattaa tacattacgg tcgttaagca aatcattttt attctacgga cagtagatat 1140

tgcaataata gttagaaaag ttggtaaaga attagtacag ttggtcaaac actattatta 1200

ttattattat tattgttatt attattatta ttctataaat ctgataataa aaacaaatag 1260

catatgaata atagcatata aatgctggat ccgatttaat tttaatttta gttaaatagc 1320

aactttcagt aaattttttt agttaactgc ctctggaaaa atgaaatgcg tgggtgggag 1380

cggttcggat ttggtacgga aaatgctttt ctccgcattg tgcggataaa tctggaccgt 1440

gcgatcaggc ccaaaattaa tccagtggtg aaggacgaga gggggtaaat ctggaatttt 1500

ttttttactc ataataaaaa acgattatat cttttgaatg ccaaaacaaa ataaaaaaat 1560

aaaaacacca aatttcatca tttttaattc cctacaaaaa gagatccatc ttgctatata 1620

aattttatat ttttagggtt tagggtttag ggtttacggc ctggcacaga cacatctaaa 1680

acgaagcaca actcagctaa acgaagcaca aattcattat tatgaagcac atattcatta 1740

gtatgaagca catattcatt tttatgaagc gcaattcatt tgtatgaagc acatattcat 1800

ttttatgaag cgcaattcat ttgtatgaag cacaaaatca tttgtatgaa acttataatc 1860

atttgtatga agcacaaatt catttatatg aagcaaaaat tcatttgtat gaacgcaatt 1920

catttgtatg aatcacaaat taatttgtat gaagcacaaa ttaatttgta tgaagcacaa 1980

ctcatttgta caaaatcata caaataatta taagtaccat ctaattgaag cacaaattca 2040

tttgtttgta tgaagcacaa attcatttgt ataaagcaca aattcatttg tacaatacat 2100

ccaaataata tataaagcac catctaaacg aagtacaaat tcatttgtat gaagcacaaa 2160

ttcgtacaaa aaatatttaa agcacaatct aaaacgaagc acagattcat ttgtatgaag 2220

cacaaattca tttgtacaat acatccaaat aatatataaa gcaccatcta aacgaagcac 2280

aaattcattt gtatgaagca caaattcatt tgtacaaatt catacaaaaa atatttaaag 2340

cacaatctaa aacgaagcac agattcattt gtatgaagca cagattcatc aggggtattt 2400

tagacaaaaa cccgacgaat atcatccgtt agatttattt tgagcaaatc caatggccca 2460

ttacttttgt ccgcacatac ggagaaaagt accgtccgca tttgaaccag aggggatggg 2520

agggactgcg aacataatat aattcgggtc aatggcgtac caaaaaatgg tcaaataaat 2580

aatgctacaa gttaaatgca cgtgattctc ttgttgaccg ttaaatgttg atttctattt 2640

tgatcgaatt ttatagatat tagctcagct gctgagactg agagagagat tattttagaa 2700

acggatctga tcaagatcgc tcgtgattat tctcatcatc tcgtgtagca tttagatttg 2760

gtaaaaccgt aaaaatatct tgtcttaact tcttcctatt aattccacaa tctcacgcaa 2820

aaatgtattt ttacagcgac atgccttgag gaggaatttc agtgctaaag acaaaataga 2880

cttagaaatg tgccgtttga ttaattttgg tagttaaaaa aaaaaaaatt atttatttat 2940

ttatgcttag ccataataat taggacggaa acaagaagaa tttgcccatt tttaaggcct 3000

acgaatagta tacgggccat aactttatgg gggacatttt caggcctacc caatatgatt 3060

gaatgtgcct ccactccaat tatcaaaaag aaacgccgac tgagcttgag cacgattttc 3120

aaatgtctac tccacagtgc aaaaattgca cgatcaattc tgatgaaatg tattcccatc 3180

tatgtagatg aatgggagcg tttgggatcc aacactcgtt ttttctacct atttcgacgg 3240

ttttctctgg tcaggtggtt tccgagaggg gactgaaata tacagtacag ttgttgagat 3300

attgatgaga tcaatcaagt cgtgtaagct tatctttctg aaaatataat tatatttaat 3360

tgattatcct accgcttgtc aattttttct attgcgagtg tgttttggtg taaagagtag 3420

ctatcgatgc tgaagagatt tttatcaagt aagatgatgt ggagaactaa ctgattgttt 3480

gttttgttgc cagcgatata ttgatgatga tgttctttgc aattagagtt attctactta 3540

acaaactttt tctcttgtca taacatacac atagatactg catgcttttc catgattcat 3600

tctatctaat aaaggtcaat ctactcccca ctttcatcca tgccaatgaa acacatacat 3660

aaatggaaac atatcataat ttaggtgtgg ccctattagt tttcttctcg ttgaacgtcg 3720

gttgatggcc taaaaaaacg tgacaaacca cacagttgtc acccaacgta ctccattgat 3780

acacatataa agctcagtga ttgattattc ataaacactc aactggttga gcactatttg 3840

cctcttcttt gctagattca catcgagtaa gtactccgtc cgacctttta ttgtatggta 3900

taatcgtaag aaaacaaatt aagcagctag tcaccattca gt 3942

<210> 8
<211> 22
<212> DNA
<213> Artificial Sequence

<220>
<223> primer TBG10proXbaIRV

<400> 8
tctagactga atggtgacta gc 22

<210> 9
<211> 2307
<212> DNA
<213> Torenia Fournieri

<220>
<221> misc_feature
<223> Trenia F3'5'H TBG16 region in pSPB3746

<400> 9
aattaaccct cactaaaggg aacgaattcg gatcctattg cactcgtttt ttctcgacga 60

ttttctctgg tcaggtccgt tgtttggatt ccgacaggag actgaaatat acagttgttg 120

agctgctgat gagatcaatc aagtcatcta agcttatctt tctgaaatat ataattatat 180

ttaattgatt atcgtaccgc ttgtcaattt tttgtctatt gcgagtgcgt tctggtggaa 240

ttggatgtgg ccgcgatgcc tggtgtagtg tagctgaaga gattttcgag tgattgctgg 300

ttttgttgcc agcgatattg atgttctttg cagttaagag ttattctact taccaatttt 360

tctcataacg tacacaaaaa accattctat ctatctaata aaggtcaatc tactcaccac 420

tttcatccat gccataaatg gaaacatatc ataagttagg tgtgtatcgt tggataattt 480

tgccctatta cttttttttt tcctcgttga acgtcggttg atggccttaa aaaacgtgac 540

aaaccacaca gttgtcaccc aactccattg atacacatat aaagctcagt aattgattat 600

ctcaactagc tgagcattat ttgccctttc tttgctagat tcacattgtg taagtactcg 660

gaccttttgt tgtataatca gaagaaaaca aattaagcgg ctagtcacca ttcagtatga 720

cagtagctac tacacttgtc gttgtgttga gtagagagtt gttgtatgtg ttggttgtct 780

acgttctggt gcggtcaata ctcatctcaa agtcactgtc taccatcatc gtctcccgca 840

aacggttacc ccccggccca acgggcttcc ccgtggtcgg tgccctccca ttgttgggca 900

acatgcctca cgtggcgttg gccagaatgg caaaaaaata cgggcccgtc atgtacctta 960

aaacgggcac actggggatg gtggtggcct ccacgccttc gtcggcaaaa gcgttcctca 1020

agacactgga ctccaacttc tcgaaccgcc ctggcaatgc cggtgccacc cacctggcct 1080

acggggcgca ggacatggtg tttgctccct acggccccag atggagactg ctgagaaggt 1140

taagcaatct ccacatgctc ggaggcaagg ccgtggacgg atgggcaaac gtcagggcct 1200

tgagttgggg cacatgctgg aggccatgaa ccgggcgagc cgcgacgggg aggtggtggt 1260

ggtgccggag atgctggtgt acgccatggc caacatgatc gggcaagtga tactgagccg 1320

gcgggttttc gtcaccaaag gggatgaagt gaacgagttt aaggacatgg tggtggagct 1380

catgacgact gctggatact tcaacatggg cgactttgtc ccgatccttg cttggatgga 1440

cttgcaaggg attgagagag ggatgaaagc ccttcacaaa aagttggaca agctcatcac 1500

gagaatgttg gagcagcatg cggcgtcggc ccacacgccc gattttctcg acgccatcag 1560

ccttgacgac tattcgagtg gggagaggct cagtacgacc aacatcaaag ctctcttatt 1620

ggtaagcttt atatattttc cccatgccat gccatccatc ctattgaaat atgtgtataa 1680

gtggtgtaga acttgtttac tgcgggaacg gacacatcat cgagcacgat agaatgggcg 1740

ctctcggaga tgctgaaaaa ccggaaaatc ctaaggagga aatggacaga gtggtgggac 1800

gagagaggcg gctggtggaa tcggacatag aaaaactgcc gtacctggta gccatatgca 1860

aggagacttt ccggaagcat ccttcgacgc cgctgaaccg gcctcggatg tcgtcggagg 1920

cgtgcgtggt gaacggtcac tacataccca agggcacgag gctgagcgtc aacatatggg 1980

ccatcgggag agaccctgag gtgtgggagg atccgttgga atttaacccg gacaggtttc 2040

tgcactcgaa gatggatccg agggggaacg atttcgagct catgcccttc ggtgcgggtc 2100

gaaggatatg tgccgggacg aggatgggga ttgtcctcgt cgagtacata ttgggcactt 2160

tggtgcattc tttccaatgg aagctgccgc ctcatcaaga caccatcaat atggaggagg 2220

cctttgggat tgctctgcag aaggccatac ataccacttg aggctctcgt tactcctcgc 2280

cttcctttac attgttatct tccatag 2307

<210> 10
<211> 21
<212> DNA
<213> Artificial Sequence

<220>
<223> primer TBG16proFW

<400> 10
tcctattgca ctcgtttttt c 21

<210> 11
<211> 20
<212> DNA
<213> Artificial Sequence

<220>
<223> primer TBG16proRV

<400> 11
actgaatggt gactagccgc 20

<210> 12
<211> 685
<212> DNA
<213> Torenia Fournieri

<220>
<221> misc_feature
<223> Trenia 3'5'H TBG16 region in pSPB3758

<220>
<221> primer_bind

<222>   (1)..(21)
<223>   primer TBG16proFW binding

<220>
<221>   primer_bind
<222>   (666)..(685)
<223>   primer TBG16proRV binding

<400>   12
tcctattgca ctcgtttttt ctcgacgatt ttctctggtc aggtccgttg tttggattcc        60

gacaggagac tgaaatatac agttgttgag ctgctgatga gatcaatcaa gtcatctaag       120

cttatctttc tgaaatatat aattatattt aattgattat cgtaccgctt gtcaattttt       180

tgtctattgc gagtgcgttc tggtggaatt ggatgtggcc gcgatgcctg gtgtagtgta       240

gctgaagaga ttttcgagtg attgctggtt ttgttgccag cgatattgat gttctttgca       300

gttaagagtt attctactta ccaatttttc tcataacgta cacaaaaaac cattctatct       360

atctaataaa ggtcaatcta ctcaccactt tcatccatgc cataaatgga aacatatcat       420

aagttaggtg tgtatcgttg gataattttg ccctattact tttttttttt cctcgttgaa       480

cgtcggttga tggccttaaa aaacgtgaca aaccacacag ttgtcaccca actccattga       540

tacacatata aagctcagta attgattatc tcaactagct gagcattatt tgccccttct       600

ttgctagatt cacattgtgt aagtactcgg accttttgtt gtataatcag aagaaaacaa       660

attaagcggc tagtcaccat tcagt                                             685


<210>   13
<211>   24
<212>   DNA
<213>   Artificial Sequence

<220>
<223>   primer TBG16proBamHI

<400>   13
ggatccactg aatggtgact agcc                                               24


<210>   14
<211>   21
<212>   DNA
<213>   Artificial Sequence

<220>
<223>   primer TFNSR3

<400>   14
attcctaatg ggctgaaagt g                                                  21


<210>   15
<211>   4173
<212>   DNA
<213>   Torenia Fournieri

<220>
<221>  misc_feature
<223>  Trenia FNS transcriptional region in pSPB3747

<400>  15
tccttggcaa ttttattttt atttgtttta atgaagaagc gaagtacagt tccttggatc      60

ctgcaaatga aaccccatca gtgtccacaa atcatcatat atcttgtcct tcaatttcta     120

taccctacaa aggttagtcc ttcagtcttt ctcaagcaaa agtcattttc caatggctta     180

gaaagaaaaa gacataactt ttgtatgtca tcaatacatt agctactgta ggcattttga     240

cctcttcaat attagaagga aaataaaaaa agaaacaaag cacagagacc attaaagcta     300

cgtacatctc cagcttagct tggcaataat atttctaaaa taatttcttt ctccccatat     360

ttcatcgtag cagaggcctt aatttcaaga attcttacaa gggtttctac ttattgtaat     420

atcatagggt ttatttggta tttctttcct tcaagctgtt tcacatcaac atattcaaga     480

tgtttcccaa aaacacatac ctgattcctc agatttctcc tattcattct cgcgccacta     540

cttcttcagt tcttgacctg aacgaccccg agatcttgta ttgtcaccac caccaagaca     600

tgttctctgc tcacaattat atatcagctt caaataatgc tcaggctctc gagccttcga     660

tgatgttcaa tcaagatatc tcaggcctaa acattggaga tccgaagatg tcgcccgtta     720

aacactctga atgaactctc actagaacat ccaatccatt cccgatatct ccctgcacaa     780

atacgtacac gaaaggttag tcgagataca gcatagcatt tttgaggttg aattattatt     840

tttaccttca cgagagctat atgttccatc tcgtcgatct tcgatcggta gctatgagcc     900

tgaaacaccc cccatttcgt tggcagacga gtaattgctg ttctctccac cagcttttcc     960

cttttcctca tgtacctgcg gatagatact tgaataatat tatcgttgtc actcgttctt    1020

tgcagtctct atatgttgtg tcgaggcttt gagctttacc taatgagatc tgtgatcgaa    1080

acgataggga tactatgatc cgagcccaat tttctcaaat gggccatcga ggccatagaa    1140

ccatcttctc tatcgacgat agtcgagagg acagagacag gtctcaaacc agcttgcatc    1200

accaagtcca cagaggcctc ggtgtgacca gcccgacgaa gaacacctcc gtttctatac    1260

ttgagcggga acacgtggcc cggcttgttg aaatcttcgg gcttcgactc gagagaagac    1320

agagcaagta cagttttagc cctgtccgaa gctgacacgc cagtagatgt ttcagcttta    1380

gcgtcctaaa ttgtttaaag agattattgg tgagacaaaa gaaaaggtta atgctttcgg    1440

gacatgacac atctaagatc gatctacacg ggcaataagc gtagacaacg ggtaatatga    1500

ttaccactgt gatagtgaac gaaggagcag aagaatcatg ttctttctcg ggattcatca    1560

atggcagctt caatctctcg aggtcgtctc ctttcatccc gacagagacg atgcccgatc    1620

cgtgtttgat cataaaggcg atggccttct cgctcgcgtg agaagctgcc attatgagat    1680

ttccttctag gctatcgccg ctctcatcat ctacgacaat cacaaacttc aaacacgaaa    1740

aggaaatttc agagacagac gctatgggtt ttgaaattgc gtcgcaaaat caaaacagag 1800

cctgtgttag cagtaccttt ccctgtcgca gagcagatag agcattttca atggatgagt 1860

aaccctgaga tggacgatca ggatcgcctt cggcgtcact cacgaagaaa tcaacggttt 1920

cgtgagtgat ctctgcttcg agcgtttcaa agggtgtagc gggagatgac gactcgtcat 1980

caaataggga gccatttaag gttgcccgtt tcttgattga actctcatcg tcgtcagcat 2040

gagataagtc tcctgctgaa aatttaagag catgacagta gtaagtgttg agcttgcatg 2100

gtctgcttct gatgattccg aatcccaggt tggaatgcga agattttgac gttataaaca 2160

atagcgattg ctgaagacag ataatgcaat ccattgattc ttgaattgtt ttatttctat 2220

ttggatagta aatgaagagc tttgggttat atccagtaga tgcagagatt tggaaccaca 2280

tgaatgtagg aatagagaca acaaggccaa gaatcccaac tagtagataa tatcgtgaat 2340

gatatttgta aagaacgttg tattaaattc aggacactta attgatgaat cacaatcaaa 2400

taatattatt ttgcagtatc tggtacataa gtatgatata aatgataagt gccataactg 2460

tgagaatata aactataatc gtaataaaaa tttgtatagc ataaaatatt taatatttac 2520

tataaattca ataaaatcaa tgctacatga ttatgtaaga tgatgtggat tttcttaaca 2580

aaatgatttt ggcttgatcg tgatgatact attaaaaaat aatgaagttt gtctaggaca 2640

tggcattggt ttatagatag atgtcctcat acgtttattg gcacttgaca tttgtttctt 2700

tttattcttg tgaattggga tatgtggatt tataaatatt tttagaagtc tggcttgtat 2760

ttctgatact aatttttatg atataatttc tttatgaatt aattgatatt aaaggctcat 2820

ttgaagtttg cggatgattc aatgcgagca atagcgtata tattgtgatt ggttggtgca 2880

ctgcacatat aggctcgcat cgaatcatcc gccgtttgct taggaaatta taagactcat 2940

tttattaatt taaaaataga atttgggtga aaggtagacg tacggtacgg ctacggtacc 3000

taaccttcga ggcttagact aagggtaaag aaaagaccag gaaaagaaaa aatctagaat 3060

cgttaaaata ttaaatgcaa ttaaatgaaa ataaaaaagg ataaataaag atccacacca 3120

cacctatcaa ttgtggagtc taattgtttg aacttgatac aatacaatga tatctgaagt 3180

atcgtatcga aatacacacg ttatgttttt cctatttggc acatcatatt tattcttggt 3240

ttttcaaatc caagatacaa aaactcataa aatacctcag tcaccaaaaa taacactaac 3300

agatttcaaa catactgcat gattatttaa aattaaaacc gagaagtgag atgtccaaga 3360

attgtttgtt aaaaccctca aaactctaag gatattacct aataagtcgt tggatgtaat 3420

tcttatgatg atcacaaatc acacattcaa tagttcaacg caattaccga aagtagcagg 3480

gcgaaaatgt cattatcatg atcaaaggga ccatgaatgt aattgcgcaa tcagacgacg 3540

tgtcggtgaa accaaaaaat ttagaagaaa atatagctca ctcaccacca ctactcgaac 3600

ctaacaccgc attaaatatc ctacaccact tcctttctat aaagaacaca tcaacccaca 3660

aagcttttat ctcgtttaca tcgaaaccgc tatatcatta catttacaac agcgctaaaa 3720

aaatatatat aaagcatgga cacagtctta atcacactct acaccgccct gttcgtcatc 3780

accaccacct tcctcctcct cctccgccga aggggaccac cgtctccgcc cggtcctctc 3840

tccctaccca taattggcca cctccacctc ctcggcccaa gactccacca cacgttccat 3900

gaattctcac tcaaatacgg cccattgatc cagctcaagc tcggctcgat cccgtgcgtc 3960

gtggcctcga cgcccgagct cgcgagagag tttcttaaga cgaacgagct cgcgttctcc 4020

tctcgcaagc actctacggc catagacatc gtcacctacg actcgtcctt tgctttctct 4080

ccgtacggac cctactggaa gtacatcaag aaactgtgta cctacgagct gctcggagcg 4140

aggaacctcg gacactttca gcccattagg aat 4173

<210> 16
<211> 22
<212> DNA
<213> Artificial Sequence

<220>
<223> primer TFNS1proFW

<400> 16
caaatgaaac cccatcagtg tc 22

<210> 17
<211> 24
<212> DNA
<213> Artificial Sequence

<220>
<223> primer TFNS1proRV

<400> 17
   gctttatata tattttttta gcgc 24

<210> 18
<211> 3735
<212> DNA
<213> Torenia Fournieri

<220>
<221> misc_feature
<223> FNS transcriptional region in pSPB3759

<220>
<221> primer_bind
<222> (64)..(85)
<223> TFNS1proFW binding

<220>
<221> primer_bind
<222> (3712)..(3735)

<223>  TFNS1proRV binding

<400>  18
tccttggcaa ttttattttt attttgttta atgaagaagc gaagtacagt tccttggatc      60

ctgcaaatga aaccccatca gtgtccacaa atcatcatat atcttgtcct tcaatttcta     120

taccctacaa aggttagtcc ttcagtcttt ctcaagcaaa agtcattttc caatggctta     180

gaaagaaaaa gacataactt ttgtatgtca tcaatacatt agctactgta ggcattttga     240

cctcttcaat attagaagga aaataaaaaa agaaacaaag cacagagacc attaaagcta     300

cgtacatctc cagcttagct tggcaataat atttctaaaa taatttcttt ctccccatat     360

ttcatcgtag cagaggcctt aatttcaaga attcttacaa gggtttctac ttattgtaat     420

atcatagggt ttatttggta tttctttcct tcaagctgtt tcacatcaac atattcaaga     480

tgtttcccaa aaacacatac ctgattcctc agatttctcc tattcattct cgcgccacta     540

cttcttcagt tcttgacctg aacgaccccg agatcttgta ttgtcaccac caccaagaca     600

tgttctctgc tcacaattat atatcagctt caaataatgc tcaggctctc gagccttcga     660

tgatgttcaa tcaagatatc tcaggcctaa acattggaga tccgaagatg tcgcccgtta     720

aacactctga atgaactctc actagaacat ccaatccatt cccgatatct ccctgcacaa     780

atacgtacac gaaaggttag tcgagataca gcatagcatt tttgaggttg aattattatt     840

tttaccttca cgagagctat atgttccatc tcgtcgatct tcgatcggta gctatgagcc     900

tgaaacaccc cccatttcgt tggcagacga gtaattgctg ttctctccac cagcttttcc     960

cttttcctca tgtacctgcg gatagatact tgaataatat tatcgttgtc actcgttctt    1020

tgcagtctct atatgttgtg tcgaggcttt gagctttacc taatgagatc tgtgatcgaa    1080

acgataggga tactatgatc cgagcccaat tttctcaaat gggccatcga ggccatagaa    1140

ccatcttctc tatcgacgat agtcgagagg acagagacag gtctcaaacc agcttgcatc    1200

accaagtcca cagaggcctc ggtgtgacca gcccgacgaa gaacacctcc gtttctatac    1260

ttgagcggga acacgtggcc cggcttgttg aaatcttcgg gcttcgactc gagagaagac    1320

agagcaagta cagttttagc cctgtccgaa gctgacacgc cagtagatgt ttcagcttta    1380

gcgtcctaaa ttgtttaaag agattattgg tgagacaaaa gaaaaggtta atgctttcgg    1440

gacatgacac atctaagatc gatctacacg ggcaataagc gtagacaacg ggtaatatga    1500

ttaccactgt gatagtgaac gaaggagcag aagaatcatg ttctttctcg ggattcatca    1560

atggcagctt caatctctcg aggtcgtctc ctttcatccc gacagagacg atgcccgatc    1620

cgtgtttgat cataaaggcg atggccttct cgctcgcgtg agaagctgcc attatgagat    1680

ttccttctag gctatcgccg ctctcatcat ctacgacaat cacaaacttc aaacacgaaa    1740

aggaaatttc agagacagac gctatgggtt ttgaaattgc gtcgcaaaat caaaacagag    1800

cctgtgttag cagtaccttt ccctgtcgca gagcagatag agcattttca atggatgagt 1860
aaccctgaga tggacgatca ggatcgcctt cggcgtcact cacgaagaaa tcaacggttt 1920
cgtgagtgat ctctgcttcg agcgtttcaa agggtgtagc gggagatgac gactcgtcat 1980
caaataggga gccatttaag gttgcccgtt tcttgattga actctcatcg tcgtcagcat 2040
gagataagtc tcctgctgaa aatttaagag catgacagta gtaagtgttg agcttgcatg 2100
gtctgcttct gatgattccg aatcccaggt tggaatgcga agattttgac gttataaaca 2160
atagcgattg ctgaagacag ataatgcaat ccattgattc ttgaattgtt ttatttctat 2220
ttggatagta aatgaagagc tttgggttat atccagtaga tgcagagatt tggaaccaca 2280
tgaatgtagg aatagagaca acaaggccaa gaatcccaac tagtagataa tatcgtgaat 2340
gatatttgta aagaacgttg tattaaattc aggacactta attgatgaat cacaatcaaa 2400
taatattatt ttgcagtatc tggtacataa gtatgatata aatgataagt gccataactg 2460
tgagaatata aactataatc gtaataaaaa tttgtatagc ataaaatatt taatatttac 2520
tataaattca ataaaatcaa tgctacatga ttatgtaaga tgatgtggat tttcttaaca 2580
aaatgatttt ggcttgatcg tgatgatact attaaaaaat aatgaagttt gtctaggaca 2640
tggcattggt ttatagatag atgtcctcat acgtttattg gcacttgaca tttgtttctt 2700
tttattcttg tgaattggga tatgtggatt tataaatatt tttagaagtc tggcttgtat 2760
ttctgatact aatttttatg atataatttc tttatgaatt aattgatatt aaaggctcat 2820
ttgaagtttg cggatgattc aatgcgagca atagcgtata tattgtgatt ggttggtgca 2880
ctgcacatat aggctcgcat cgaatcatcc gccgtttgct taggaaatta taagactcat 2940
tttattaatt taaaaataga atttgggtga aaggtagacg tacggtacgg ctacggtacc 3000
taaccttcga ggcttagact aagggtaaag aaaagaccag gaaaagaaaa aatctagaat 3060
cgttaaaata ttaaatgcaa ttaaatgaaa ataaaaaagg ataaataaag atccacacca 3120
cacctatcaa ttgtggagtc taattgtttg aacttgatac aatacaatga tatctgaagt 3180
atcgtatcga aatacacacg ttatgttttt cctatttggc acatcatatt tattcttggt 3240
ttttcaaatc caagatacaa aaactcataa aatacctcag tcaccaaaaa taacactaac 3300
agatttcaaa catactgcat gattatttaa aattaaaacc gagaagtgag atgtccaaga 3360
attgtttgtt aaaaccctca aaactctaag gatattacct aataagtcgt tggatgtaat 3420
tcttatgatg atcacaaatc acacattcaa tagttcaacg caattaccga aagtagcagg 3480
gcgaaaatgt cattatcatg atcaaaggga ccatgaatgt aattgcgcaa tcagacgacg 3540
tgtcggtgaa accaaaaaat ttagaagaaa atatagctca ctcaccacca ctactcgaac 3600
ctaacaccgc attaaatatc ctacaccact tcctttctat aaagaacaca tcaacccaca 3660
aagcttttat ctcgtttaca tcgaaaccgc tatatcatta catttacaac agcgctaaaa 3720

aaatatatat aaagc                                                    3735

<210>  19
<211>  28
<212>  DNA
<213>  Artificial Sequence

<220>
<223>  primer TFNS1pBamHIRV

<400>  19
ggatccgctt tatatatatt tttttagc                                        28

<210>  20
<211>  1618
<212>  DNA
<213>  Torenia Fournieri

<220>
<221>  misc_feature
<223>  Trenia F3'5'H trancriptional region in pSPB3791

<400>  20
aatatttaaa gcacaatcta aaacgaagca cagattcatt tgtatgaagc acagattcat      60

caggggtatt ttagacaaaa acccgacgaa tatcatccgt tagatttatt ttgagcaaat     120

ccaatggccc attacttttg tccgcacata cggagaaaag taccgtccgc atttgaacca     180

gaggggatgg gagggactgc gaacataata taattcgggt caatggcgta ccaaaaaatg     240

gtcaaataaa taatgctaca agttaaatgc acgtgattct cttgttgacc gttaaatgtt     300

gatttctatt ttgatcgaat tttatagata ttagctcagc tgctgagact gagagagaga     360

ttattttaga aacggatctg atcaagatcg ctcgtgatta ttctcatcat ctcgtgtagc     420

atttagattt ggtaaaaccg taaaaatatc ttgtcttaac ttcttcctat taattccaca     480

atctcacgca aaaatgtatt tttacagcga catgccttga ggaggaattt cagtgctaaa     540

gacaaaatag acttagaaat gtgccgtttg attaattttg gtagttaaaa aaaaaaaaat     600

tatttattta tttatgctta gccataataa ttaggacgga aacaagaaga atttgcccat     660

ttttaaggcc tacgaatagt atacgggcca taactttatg ggggacattt tcaggcctac     720

ccaatatgat tgaatgtgcc tccactccaa ttatcaaaaa gaaacgccga ctgagcttga     780

gcacgatttt caaatgtcta ctccacagtg caaaaattgc acgatcaatt ctgatgaaat     840

gtattcccat ctatgtagat gaatgggagc gtttgggatc caacactcgt tttttctacc     900

tatttcgacg gttttctctg gtcaggtggt ttccgagagg ggactgaaat atacagtaca     960

gttgttgaga tattgatgag atcaatcaag tcgtgtaagc ttatctttct gaaaatataa    1020

ttatatttaa ttgattatcc taccgcttgt caattttttc tattgcgagt gtgttttggt    1080

gtaaagagta gctatcgatg ctgaagagat ttttatcaag taagatgatg tggagaacta 1140

actgattgtt tgttttgttg ccagcgatat attgatgatg atgttctttg caattagagt 1200

tattctactt aacaaacttt ttctcttgtc ataacataca catagatact gcatgctttt 1260

ccatgattca ttctatctaa taaaggtcaa tctactcccc actttcatcc atgccaatga 1320

aacacataca taaatggaaa catatcataa tttaggtgtg gccctattag ttttcttctc 1380

gttgaacgtc ggttgatggc ctaaaaaaac gtgacaaacc acacagttgt cacccaacgt 1440

actccattga tacacatata aagctcagtg attgattatt cataaacact caactggttg 1500

agcactattt gcctcttctt tgctagattc acatcgagta agtactccgt ccgacctttt 1560

attgtatggt ataatcgtaa gaaaacaaat taagcagcta gtcaccattc agtctaga 1618

<210> 21
<211> 1416
<212> DNA
<213> Torenia Fournieri

<220>
<221> misc_feature
<223> Trenia FNS transcriptional region in pSPB3789

<400> 21
ctagtagata atatcgtgaa tgatatttgt aaagaacgtt gtattaaatt caggacactt 60

aattgatgaa tcacaatcaa ataatattat tttgcagtat ctggtacata agtatgatat 120

aaatgataag tgccataact gtgagaatat aaactataat cgtaataaaa atttgtatag 180

cataaaatat ttaatattta ctataaattc aataaaatca atgctacatg attatgtaag 240

atgatgtgga ttttcttaac aaaatgattt tggcttgatc gtgatgatac tattaaaaaa 300

taatgaagtt tgtctaggac atggcattgg tttatagata gatgtcctca tacgtttatt 360

ggcacttgac atttgtttct ttttattctt gtgaattggg atatgtggat ttataaatat 420

ttttagaagt ctggcttgta tttctgatac taatttttat gatataattt ctttatgaat 480

taattgatat taaaggctca tttgaagttt gcggatgatt caatgcgagc aatagcgtat 540

atattgtgat tggttggtgc actgcacata taggctcgca tcgaatcatc cgccgtttgc 600

ttaggaaatt ataagactca ttttattaat ttaaaaatag aatttgggtg aaaggtagac 660

gtacggtacg gctacggtac ctaaccttcg aggcttagac taagggtaaa gaaaagacca 720

ggaaaagaaa aaatctagaa tcgttaaaat attaaatgca attaaatgaa aataaaaaag 780

gataaataaa gatccacacc acacctatca attgtggagt ctaattgttt gaacttgata 840

caatacaatg atatctgaag tatcgtatcg aaatacacac gttatgtttt tcctatttgg 900

cacatcatat ttattcttgg tttttcaaat ccaagataca aaaactcata aaatacctca 960

gtcaccaaaa ataacactaa cagatttcaa acatactgca tgattattta aaattaaaac 1020

cgagaagtga gatgtccaag aattgtttgt taaaaccctc aaaactctaa ggatattacc 1080

taataagtcg ttggatgtaa ttcttatgat gatcacaaat cacacattca atagttcaac 1140

gcaattaccg aaagtagcag ggcgaaaatg tcattatcat gatcaaaggg accatgaatg 1200

taattgcgca atcagacgac gtgtcggtga aaccaaaaaa tttagaagaa aatatagctc 1260

actcaccacc actactcgaa cctaacaccg cattaaatat cctacaccac ttcctttcta 1320

taaagaacac atcaacccac aaagctttta tctcgtttac atcgaaaccg ctatatcatt 1380

acatttacaa cagcgctaaa aaaatatata taaagc 1416